# EUROPÄISCHE PATENTANMELDUNG

(11) **EP 4 603 072 A1**
(43) Veröffentlichungstag der Anmeldung: **20.08.2025**
(21) Anmeldenummer: 24157901.0
(22) Anmeldetag: 15.02.2024
(51) Int. Cl.: A61F 13/02, A61F 13/0246, A61F 13/05, A61F 13/00

(54) **FILMVERBAND FÜR EINE WUNDBEHANDLUNG**

(71) Anmelder: Paul Hartmann AG, 89522 Heidenheim (DE)
(72) Erfinder: CROIZAT, Pierre, 89542 Herbrechtingen (DE); ECKSTEIN, Axel, verstorben (DE)
(74) Vertreter: Paul Hartmann AG Patents & Licensing

(57) **Zusammenfassung**

Die vorliegende Erfindung betrifft einen transparenten Filmverband für eine Wundbehandlung. Der Filmverband weist an seiner Unterseite einen stärker klebenden Randbereich mit einem Acrylatklebstoff und einen schwächer klebenden zentralen Bereich mit einem Silikongel auf. Der erfindungsgemäße Filmverband kann gut an der Wundstelle haften und weist atraumatische Eigenschaften auf. Der erfindungsgemäße Filmverband kann auch vorteilhaft als Wundabdeckung in der Unterdruckwundtherapie verwendet werden.

## Beschreibung

Die vorliegende Erfindung betrifft einen Filmverband für eine Wundbehandlung. Die vorliegende Erfindung betrifft zudem einen Kit sowie ein System für die Unterdruckwundtherapie mit dem Filmverband. Es wird auch ein Verfahren zur Herstellung des Filmverbands angegeben.

Filmverbände für die Wundbehandlung sind im Stand der Technik bekannt. Sie werden für die Behandlung von trockenen oder nur leicht exsudierenden, primär heilenden Wunden, fast verheilten epithelisierenden Wunden sowie zur Fixierung von Wundauflagen, Kathetern und Kanülen verwendet. Darüber hinaus werden Filmverbände auch als abdichtende Wundabdeckung in der Unterdruckwundtherapie in Kombination mit mindestens einer weiteren Verbandslage, zum Beispiel einem offenzelligen Schaumstoff als Wundfüllmaterial, eingesetzt. Filmverbände in dem vorliegend gemeinten Sinne enthalten keine absorbierenden Verbandsschichten. Daher sind sie allein nicht zur Behandlung von stärker blutenden oder exsudierenden Wunden geeignet.

Ein üblicher Filmverband ist einfach aufgebaut und umfasst als wesentliche Verbandskomponente eine einseitig adhäsiv beschichtete, flüssigkeitsundurchlässige und wasserdampfdurchlässige Kunststofffolie. Zusätzlich kann der Filmverband Stütz- und Schutzfolien als Applikationshilfe umfassen, welche lösbar an der nicht-klebenden Oberseite beziehungsweise der klebenden Unterseite der Kunststofffolie befestigt sind und vom Anwender entfernt werden, wenn der Filmverband am Körper des Patienten befestigt wird.

Die adhäsive Schicht des Filmverbands kann zum Beispiel aus einem Acrylatklebstoff bestehen. Acrylatklebstoffe können eine hohe Klebkraft aufweisen. Somit kann der Filmverband gut und dauerhaft am Körper des Patienten haften und zum luftdichten Verschließen eines Unterdruckwundtherapieverbands verwendet werden. Die hohe Klebkraft des Acrylatklebstoffs kann allerdings in manchen Fällen zu Schmerzen und zur Beschädigung der Wundoberfläche sowie gegebenenfalls sogar von intakter Haut in der Wundumgebung führen, wenn der Filmverband wieder vom Körper des Patienten beispielsweise im Rahmen eines Verbandswechsels entfernt wird.

Aufgabe der vorliegenden Erfindung war es, einen verbesserten Filmverband für die Wundbehandlung anzugeben. Der Filmverband sollte dabei möglichst gut und dauerhaft am Körper des Patienten haften und zum luftdichten Verschließen eines Unterdruckwundtherapieverbands verwendet werden können. Weiterhin sollte der Filmverband möglichst keine oder zumindest weniger Schmerzen und Schäden an der Haut- und Wundoberfläche verursachen, wenn der Filmverband wieder vom Körper des Patienten entfernt wird. Die genannten Aufgaben wurden mit einem Filmverband nach Anspruch 1, einem Kit für eine Unterdruckwundtherapie nach Anspruch 13, einem System für eine Unterdruckwundtherapie nach Anspruch 14 und einem Herstellungsverfahren nach Anspruch 15 gelöst.

Der erfindungsgemäße Filmverband umfasst mehrere Folienschichten sowie mehrere adhäsive Schichten, welche nachfolgend angegeben sind:
i. **Eine erste, flüssigkeitsundurchlässige und wasserdampfdurchlässige Kunststofffolie** mit einer Oberseite und einer Unterseite. Die erste Kunststofffolie bildet die Rückschicht des Filmverbands, also die äußerste Schicht des Filmverbands, wenn der Filmverband am Körper des Patienten befestigt ist.
ii. **Eine erste adhäsive Schicht** mit einer Oberseite und einer Unterseite. Die erste adhäsive Schicht ist mit ihrer gesamten Oberseite direkt an der Unterseite der ersten Kunststofffolie angeordnet und besteht aus einem Acrylatklebstoff.
iii. **Eine zweite adhäsive Schicht** mit einer Oberseite und einer Unterseite. Die zweite adhäsive Schicht ist mit ihrer gesamten Oberseite direkt an der Unterseite der ersten adhäsiven Schicht angeordnet und besteht aus einem Acrylatklebstoff.
iv. **Eine zweite Kunststofffolie** mit einer Oberseite und einer Unterseite. Die zweite Kunststofffolie ist mit ihrer gesamten Oberseite direkt an der Unterseite der zweiten adhäsiven Schicht angeordnet.
v. **Eine dritte adhäsive Schicht** mit einer Oberseite und einer Unterseite. Die dritte adhäsive Schicht ist mit ihrer gesamten Oberseite direkt an der Unterseite der zweiten Kunststofffolie angeordnet und besteht aus einem Silikongel.

Dabei bildet die erste Kunststofffolie mit der ersten adhäsiven Schicht einen ersten Verbund aus. Die zweite Kunststofffolie bildet wiederum mit der zweiten und dritten adhäsiven Schicht einen zweiten Verbund aus.

Weitere wesentliche Merkmale des erfindungsgemäßen Filmverbands sind, dass der erste Verbund den zweiten Verbund überragt und der zweite Verbund perforiert ist. Indem der erste Verbund den zweiten Verbund überragt, weist der Filmverband einen adhäsiven Rand bestehend aus der ersten Kunststofffolie und der ersten adhäsiven Schicht auf. Die Perforationen in dem zweiten Verbund sind als durchgehende Perforationen ausgebildet. Das heißt, die Perforationen durchdringen alle drei Schichten des zweiten Verbunds (zweite adhäsive Schicht, zweite Kunststofffolie und dritte adhäsive Schicht) gleichermaßen, so dass Flüssigkeiten und Gase den zweiten Verbund leicht passieren können. So können die Perforationen in dem zweiten Verbund die Wasserdampfdurchlässigkeit (Atmungsaktivität) des Filmverbands verbessern.

Die Begriffe Film und Folie werden im vorliegenden Dokument synonym verwendet. Der vorliegende Filmverband könnte daher auch als Folienverband oder die erste Kunststofffolie auch als erster Kunststofffilm bezeichnet werden.

Eine Oberseite einer Schicht des Filmverbands ist bei bestimmungsgemäßem Gebrauch des Filmverbands der Haut beziehungsweise der Wunde abgewandt. Entsprechend ist eine Unterseite einer Schicht des Filmverbands bei bestimmungsgemäßem Gebrauch des Filmverbands der Haut beziehungsweise der Wunde zugewandt.

Eine Seite einer Schicht ist im Sinne der vorliegenden Erfindung dann direkt an einer Seite einer anderen Schicht angelagert, wenn die Seiten der beiden Schichten unmittelbaren Kontakt miteinander haben und sich damit gegenseitig berühren. Die Seiten der beiden Schichten dürfen folglich nicht durch eine Zwischenschicht voneinander beabstandet beziehungsweise getrennt sein.

Die zur Anbringung an der Haut- beziehungsweise Wundoberfläche vorgesehene Unterseite des in der Beschreibungseinleitung genannten Filmverbands aus dem Stand der Technik wird ausschließlich von einem stark haftenden Acrylatklebstoff gebildet. Im Unterschied dazu weist die Unterseite des vorliegenden Filmverbands eine vorteilhafte Klebstoffkombination aus einem stärker haftenden Acrylatklebstoff im Randbereich und einem atraumatischen, schwächer haftenden Silikongel im zentralen Bereich auf. Durch den stärker klebenden Rand mit dem Acrylatklebstoff kann der Filmverband gut und dauerhaft am Körper des Patienten haften und zum luftdichten Verschließen eines Unterdruckwundtherapieverbands verwendet werden. Daher werden zusätzliche Mittel zum Befestigen oder Abdichten des Filmverbands wie zum Beispiel die aus der EP 0 782 421 B1 bekannten Hydrogelstreifen, Klebestreifen, Spulenpflaster oder ähnliches in der Regel nicht benötigt. Durch den atraumatischen, schwächer klebenden zentralen Bereich mit dem Silikongel verursacht der Filmverband keine oder zumindest weniger Schmerzen und Schäden an der Haut- und Wundoberfläche, wenn der Filmverband wieder vom Körper des Patienten entfernt wird. Darüber hinaus lässt sich der erfindungsgemäße Filmverband auch vergleichsweise leicht herstellen. Weitere Vorteile des vorliegenden Filmverbands ergeben sich aus den nachfolgend beschriebenen bevorzugten Ausführungsformen.

Bevorzugt sind die erste und zweite Kunststofffolie sowie die erste bis dritte adhäsive Schicht im Wesentlichen transparent ausgebildet. Dadurch kann die Haut- und Wundoberfläche ohne Abziehen des Filmverbands von der Wundstelle begutachtet werden. Damit können zum Beispiel unnötige Verbandswechsel vermieden werden.

Die erste Kunststofffolie kann mit Ausnahme der noch nachfolgend beschriebenen Öffnung im Filmverband als geschlossene, durchgängige Schicht ausgebildet sein. Weiterhin besteht die erste Kunststofffolie bevorzugt aus Polyurethan. Die Dicke der ersten Kunststofffolie kann 10 µm bis 100 µm, bevorzugt 15 µm bis 35 µm und insbesondere etwa 20 µm betragen.

Die erste adhäsive Schicht kann gleichfalls abgesehen von der noch nachfolgend beschriebenen Öffnung im Filmverband als geschlossene, durchgängige Schicht ausgebildet sein. Sie wird typischerweise als Beschichtung, insbesondere als vollflächige Beschichtung, auf die erste Kunststofffolie aufgetragen. Dabei stellt die erste Kunststofffolie das Trägermaterial für die erste adhäsive Schicht dar. Denkbar ist auch, dass die erste adhäsive Schicht gemustert, beispielsweise gitterförmig oder streifenförmig, ausgebildet ist (Musterbeschichtung). Damit kann die Wasserdampfdurchlässigkeit des Filmverbands verbessert werden. Zudem kann die Dicke der ersten adhäsiven Schicht 20 µm bis 60 µm betragen.

Die Klebkraft der ersten adhäsiven Schicht kann mehr als 2,8 N/25 mm, bevorzugt mehr als 3,5 N/25 mm, und besonders bevorzugt mehr als 4 N/25 mm betragen. Damit der Filmverband nicht zu stark an der Wundstelle haftet, beträgt die Klebkraft der ersten adhäsiven Schicht bevorzugt nicht mehr als 12 N/25 mm. Die Klebkraft der ersten adhäsiven Schicht kann nach DIN EN ISO 29862:2019 (Verfahren 1) bestimmt werden, wobei in folgenden Punkten von der Norm abgewichen werden kann:
- Als Substrat werden Polypropylen-Platten (PP-Platten) anstatt Stahlplatten verwendet. Die PP-Platten können Abmessungen von 150 mm x 50 mm x 4 mm aufweisen und als Einwegprüfkörper ausgebildet sein, deren Prüfseite mit Folie abgedeckt ist. Die Folie wird vor dem Test entfernt. Die PP-Platten können im Hinblick auf ihre Oberflächenbeschaffenheit den in der Norm vorgesehenen Stahlplatten entsprechen. Entsprechend sind die PP-Platten daher ebenso typischerweise vollständig eben ausgebildet und weisen eine glatte (Prüf)Oberfläche auf.
- Die Probenbreite ist 25 mm anstatt 24 mm.
- Die Wartezeit ist 20 min anstatt < 1 min (das heißt, die Probe wird erst 20 min nachdem sie auf die PP-Platte aufgebracht wurde vermessen).
- Gegebenenfalls wird ein Verlängerungstape verwendet, falls die Probe zu kurz ist (zum Beispiel das Tape Scotch 8959).
- Gegebenenfalls wird ein Verstärkungstape verwendet, falls die Probe elastisch ist (zum Beispiel das Tape Tesa 4104).
Für die Bestimmung der Klebkraft der ersten adhäsiven Schicht kann eine Zugprüfmaschine gemäß DIN EN ISO 7500-01:2018-06, Klasse 1 verwendet werden. Die Auswertung der Messergebnisse kann nach DIN ISO 6133:2017-04 (Verfahren B) erfolgen.

Die Dicke der zweiten adhäsiven Schicht kann 30 µm bis 50 µm betragen. Die Klebkraft der zweiten adhäsiven Schicht kann 2 N/25 mm bis 7 N/25 mm, insbesondere etwa 4,5 N/25 mm, betragen. Die Klebkraft der zweiten adhäsiven Schicht kann nach FINAT Nr. 1 (FTM 1 Peel adhesion (90°) at 300 mm per minute) bestimmt werden, wobei in folgenden Punkten von der Norm abgewichen werden kann:
- Als Substrat wird eine Stahlplatte anstatt Glas verwendet. Die Stahlplatte kann eine Abmessung von 50 mm x 150 mm (Breite x Länge) aufweisen und ist typischerweise vollständig eben und glatt ausgebildet.
- Die Kontroll-Atmosphäre ist 20 ± 2 °C, 60 ± 5 % rF oder 23 ± 2 °C, 50 ± 5 % rF.
- Die Messungen werden unmittelbar nach der Vorbereitung der Probenstücke durchgeführt.

Ebenso wie die erste Kunststofffolie besteht die zweite Kunststofffolie bevorzugt aus Polyurethan. Wie später noch deutlich wird, bildet die zweite Kunststofffolie im Herstellungsprozess des vorliegenden Filmverbands typischerweise das Trägermaterial für die zweite und dritte adhäsive Schicht, welche als Beschichtungen auf die beiden Seiten der zweiten Kunststofffolie aufgetragen werden. Der dabei gebildete dreischichtige Verbund wird anschließend perforiert, damit der Verbund durchlässig für Flüssigkeiten und Gase wird. Die Dicke der zweiten Kunststofffolie kann 20 µm bis 30 µm, zum Beispiel etwa 25 µm, betragen.

Erfindungsgemäß besteht die dritte adhäsive Schicht aus einem Silikongel. Silikongele für Wundauflagen sind den Fachmann ebenso wie Acrylatklebstoffe bekannt und zum Beispiel in der EP 4 218 698 A1 näher beschrieben. Die Dicke der dritten adhäsiven Schicht kann 80 µm bis 200 µm, vorzugsweise 100 µm bis 150 µm, betragen.

Das Silikongel der dritten adhäsiven Schicht hat eine geringere Klebkraft als der Acrylatklebstoff der ersten adhäsiven Schicht. Entsprechend beträgt die Klebkraft der dritten adhäsiven Schicht vorzugsweise nur 1,5 N/25 mm bis 2,7 N/25 mm. Die Klebkraft der dritten adhäsiven Schicht kann nach FINAT Nr. 1 (FTM 1 Peel adhesion (180°) at 300 mm per minute) bestimmt werden, wobei in folgenden Punkten von der Norm abgewichen werden kann:
- Als Substrat wird Bristolpapier anstatt Glas verwendet. Bei dem Bristolpapier kann es sich zum Beispiel um Oxford, unifarbenes weißes Papier, nicht perforiert, Referenz 237000 handeln.
- Die Kontroll-Atmosphäre ist 20 ± 2 °C, 60 ± 5 % rF oder 23 ± 2 °C, 50 ± 5 % rF.
- Die Messungen werden innerhalb von 20 Minuten nach der Probenherstellung durchgeführt.

Wie bereits beschrieben bildet die erste Kunststofffolie mit der ersten adhäsiven Schicht einen ersten Verbund und die zweite Kunststofffolie mit der zweiten und dritten adhäsiven Schicht einen zweiten Verbund aus. Die in dem ersten Verbund enthaltenen Schichten, nämlich die erste Kunststofffolie und die erste adhäsive Schicht, sind üblicherweise deckungsgleich ausgebildet und liegen exakt (bündig) aufeinander. Entsprechendes gilt für den zweiten Verbund. Somit sind die in dem zweiten Verbund enthaltenen Schichten, nämlich die zweite Kunststofffolie, die zweite adhäsive Schicht und die dritte adhäsive Schicht, gleichfalls üblicherweise deckungsgleich ausgebildet und liegen exakt (bündig) aufeinander.

Der erste Verbund ist im Hinblick auf seine Fläche größer als der zweite Verbund ausgebildet und kann deshalb den zweiten Verbund seitlich überragen, wobei der bereits erwähnte adhäsive Rand des Filmverbands gebildet wird. Der adhäsive Rand ist dafür vorgesehen an intakter Haut, nicht hingegen an der Wundoberfläche oder Wundrändern befestigt zu werden. Der adhäsive Rand gewährleistet den sicheren Halt des Filmverbands am Körper des Patienten. Der adhäsive Rand kann zum Beispiel eine Breite von 1,5 cm bis 5 cm aufweisen.

Der zweite Verbund ist normalerweise im Wesentlichen zentral an dem ersten Verbund angeordnet und normalerweise derart dimensioniert, dass der adhäsive Rand den zweiten Verbund vollumfänglich umgeben kann. Der zweite Verbund ist damit im Hinblick auf seine Position ähnlich wie das absorbierende Wundkissen bei einem klassischen Inselverband an der adhäsiven Rückschicht befestigt. Ferner kann der zweite Verbund ein Flächengewicht von 150 g/m² bis 200 g/m² aufweisen. Dabei können 70 g/m² bis 130 g/m² auf das Silikongel entfallen. Zudem kann der zweite Verbund beziehungsweise die dritte adhäsive Schicht des zweiten Verbunds 30 % bis 90 %, bevorzugt 30 % bis 80 %, mehr bevorzugt 30 % bis 70 % und besonders bevorzugt 50 % bis 70 % einer Unterseite des Filmverbands bilden, wobei auf die klebende Unterseite des Filmverbands ohne optionale Schutzschicht abgestellt wird. Außerdem kann dabei die durch die Perforationen erhaltene offene Fläche in dem zweiten Verbund unberücksichtigt bleiben. Bei einem kleineren Anteil an silikonisierter Fläche ist die Haftkraft des Filmverbands höher. Bei einem größeren Anteil an silikonisierter Fläche ist die Haftkraft des Filmverbands niedriger. Eine höhere Haftkraft (und damit ein größerer adhäsiver Randbereich) kann vorteilhaft sein, wenn der Filmverband an unebenen oder beweglichen Körperstellen (zum Beispiel Ellbogen, Knie) oder für die Unterdruckwundtherapie verwendet werden soll. Eine niedrigere Haftkraft (und damit kleinerer adhäsiver Randbereich) kann vorteilhaft sein, wenn der Filmverband an besonders empfindlicher Haut verwendet werden soll.

Die Perforationen in dem zweiten Verbund können insbesondere im Wesentlichen rund ausgestaltet sein. Ihr Durchmesser kann dann 0,5 mm bis 3 mm, bevorzugt 0,5 mm bis 2 mm, mehr bevorzugt 0,5 mm bis 1,5 mm und besonders bevorzugt 0,5 mm bis 1 mm betragen. Außerdem sind die Perforationen vorzugsweise über die gesamte Fläche des zweiten Verbunds in regelmäßigen Abständen verteilt vorhanden. Die Perforationen können in den zweiten Verbund geschnitten oder gestanzt sein.

Der Filmverband umfasst in der Regel weiterhin mindestens eine Schutzfolie, bevorzugt zwei Schutzfolien, mit jeweils einer Oberseite und einer Unterseite. Jede Schutzfolie ist mit ihrer Oberseite direkt an der Unterseite der ersten adhäsiven Schicht im Bereich des adhäsiven Rands und der dritten adhäsiven Schicht lösbar angeordnet. Die Schutzfolien schützen die klebende Unterseite des Filmverbands vor Kontamination und erleichtert die Handhabung des Filmverbands. Sie werden vom Anwender entfernt, wenn der Filmverband am Körper des Patienten befestigt wird. Um den Filmverband nach dem Entfernen der Schutzfolien zu stabilisieren und damit die Handhabung des Filmverbands zu verbessern, kann der Filmverband weiterhin mindestens eine Stützfolie, bevorzugt zwei Stützfolien, mit jeweils einer Oberseite und einer Unterseite umfassen. Jede Stützfolie ist mit ihrer Unterseite direkt an der Oberseite der ersten Kunststofffolie lösbar angeordnet. Ebenso wie die zuvor genannten Schutzfolien werden die Stützfolien vom Anwender entfernt, wenn der Filmverband am Körper des Patienten befestigt wird. Sowohl die Schutzfolien als auch die Stützfolien können aus einem Kunststoffmaterial oder Papier, insbesondere silikonisiertem Papier, bestehen. Letztendlich handelt es sich bei den Schutzfolien und den Stützfolien also um Abziehfolien, welche eine Applikationshilfe beziehungsweise ein Applikationssystem für den Filmverband bilden.

Um die Schutzfolien von den Stützfolien und damit auch die Unterseite von der Oberseite des Filmverbands leichter unterscheiden zu können, weisen die Schutz- und Stützfolien vorteilhafterweise Beschriftungen und/oder Markierungen, insbesondere farbige Markierungen, auf. Zum Beispiel können bei einem Applikationssystem mit jeweils zwei Schutz- und Stützfolien die beiden Schutzfolien mit den Nummern 1 und 2 beschriftet und die beiden Stützfolien mit den Nummern 3 und 4 beschriftet sein. Eine solche Nummerierung würde vorteilhafterweise auch gleichzeitig zum Ausdruck bringen, dass die Schutzfolien zuerst vom Filmverband entfernt werden sollen. Zudem können die Schutzfolien und/oder Stützfolien eine Markierung für die Position beziehungsweise die Breite des adhäsiven Rands aufweisen. Damit kann die bestimmungsgemäße Befestigung des Filmverbands an der Wundstelle vereinfacht werden.

Der vorliegende Filmverband selbst enthält wie bei transparenten Post-OP-Verbänden üblich kein Wundkissen zur Absorption von Flüssigkeit oder zum Verteilen von Flüssigkeit oder Unterdruck. Typischerweise besteht der vorliegende Filmverband aus den folgenden, bereits zuvor beschriebenen Komponenten:
i. der ersten Kunststofffolie
ii. der ersten adhäsiven Schicht
iii. der zweiten adhäsiven Schicht
iv. der zweiten Kunststofffolie
v. der dritten adhäsiven Schicht
vi. optional der mindestens einen Schutzfolie
vii. optional der mindestens einen Stützfolie

Ein Kontakt der ersten adhäsiven Schicht mit der Haut- oder Wundoberfläche durch die Perforationen in dem zweiten Verbund kann die atraumatischen Eigenschaften des Filmverbands beeinträchtigen und sollte daher vorteilhafterweise verhindert oder zumindest so weit wie möglich minimiert werden. Entsprechend ist der Filmverband vorzugsweise derart ausgebildet, dass lediglich die erste adhäsive Schicht im Bereich des adhäsiven Rands und die dritte adhäsive Schicht mit einer Haut- oder Wundoberfläche eines Patienten in Kontakt treten kann, wenn der Filmverband am Körper des Patienten befestigt ist. Die nachfolgende bevorzugte Ausführungsform der Erfindung beschreibt genauer, wie dies erreicht werden kann.

In einer bevorzugten Ausführungsform der Erfindung ist der Filmverband derart ausgebildet, dass die erste adhäsive Schicht nicht in die Perforationen des zweiten Verbunds bis zur Unterseite der dritten adhäsiven Schicht eindringen kann, wenn Druck auf die Oberseite der ersten Kunststofffolie ausgeübt wird. Andernfalls könnte das erste Adhäsiv durch die Perforationen hindurchgedrückt werden und an der Haut- und Wundoberfläche anhaften. Dadurch könnten wie bereits erwähnt die vorteilhaften und erwünschten atraumatischen Eigenschaften des Filmverbands beeinträchtigt werden. Ob die erste adhäsive Schicht in die Perforationen des zweiten Verbunds bis zur Unterseite der dritten adhäsiven Schicht eindringen kann, wird insbesondere von der Dehnbarkeit und Flexibilität der ersten Kunststofffolie, der Dicke der Folienschichten, der Dicke der Klebstoffschichten und der Größe der Perforationen abhängen.

Obwohl die klebende Unterseite des vorliegenden Filmverbands teilweise silikonisiert und dadurch schwächer klebend ausgebildet ist, kann der Filmverband dennoch ähnlich wie die stark klebenden Filmverbände aus dem Stand der Technik ohne ein zusätzliches, separat bereitgestelltes Mittel zum Befestigen und/oder Abdichten des Filmverbands verwendet werden. Der Filmverband kann also dafür vorgesehen beziehungsweise ausgebildet sein, ohne ein zusätzliches, separat bereitgestelltes Mittel zum Befestigen und/oder Abdichten des Filmverbands am Körper eines Patienten für die Wundbehandlung verwendet zu werden. Wenn ein solches zusätzliches Mittel nicht benötigt wird, können unter anderem die Kosten für die Wundversorgung verringert werden.

Das allgemeine Einsatzgebiet von Filmverbänden wurde eingangs bereits beschrieben. Der vorliegende Filmverband kann vorteilhaft für die Behandlung von trockenen oder nur leicht exsudierenden, primär heilenden Wunden sowie fast verheilten epithelisierenden Wunden verwendet werden. Dabei wird der Filmverband ohne weitere Verbandsmaterialien an der Wundstelle befestigt und bildet die primäre Wundauflage. Bei dieser Anwendung wird der adhäsive Rand mit dem Acrylatklebstoff (nur) mit intakter Haut in der Wundumgebung und das Silikongel mit der Wundoberfläche in direktem Kontakt gebracht. Somit fungiert der zweite Verbund beziehungsweise das Silikongel des zweiten Verbunds bei dieser Anwendung als Wundkontaktschicht des Filmverbands.

Der erfindungsgemäße Filmverband kann auch vorteilhaft als abdichtende Wundabdeckung in der Unterdruckwundtherapie verwendet werden. Dabei wird die Wunde zunächst mit einer porösen Wundauflage zum Verteilen von Unterdruck, beispielsweise einer Gaze oder einem offenzelligen Schaumstoff, ausgefüllt. Anschließend wird der Filmverband über der (primären) Wundauflage an der Wundstelle befestigt und versiegelt die Wundstelle. Der adhäsive Rand des Filmverbands wird ebenso wie im zuvor beschriebenen Anwendungsfall (nur) an intakter Haut in der Wundumgebung befestigt und gewährleistet den sicheren Halt und die Dichtheit des Unterdrucktherapieverbands. Der atraumatische, silikonisierte Bereich des Filmverbands bedeckt dann zumindest die Wundränder und das Wundfüllmaterial. Wenngleich der Filmverband hierbei also als sekundäres Verbandsmaterial verwendet wird und folglich nicht die unmittelbare Wundkontaktlage bildet, so kann er dennoch zumindest die Wundränder sowie die Haut in der Wundumgebung vor Schäden bei einem Verbandswechsel besser schützen. Somit wird der Verbandswechsel angenehmer für den Patienten.

Damit ein Unterdruck im Wundraum hergestellt werden kann, wird typischerweise eine Öffnung in den Filmverband eingebracht. An die Öffnung wird ein Saugschlauch angeschlossen, welcher mit einer Unterdruckquelle in Verbindung steht. Die Öffnung kann von dem Anwender in den Filmverband geschnitten werden. Der Filmverband kann auch bereits herstellerseitig mit einer Öffnung zur Verfügung gestellt werden, wenn er speziell für die Anwendung in der Unterdruckwundtherapie vorgesehen ist. Entsprechend weist der Filmverband in einer Ausführungsform eine Öffnung zum Absaugen von Luft im Rahmen einer Unterdruckwundtherapie auf. Dabei durchdringt die Öffnung zumindest die erste Kunststofffolie, die erste adhäsive Schicht, die zweite adhäsive Schicht, die zweite Kunststofffolie und die dritte adhäsive Schicht. Die Öffnung kann auch Schutz- und Stützfolien durchdringen, insofern diese vorhanden sind. Die Öffnung kann zum Beispiel im Wesentlichen rund ausgestaltet sein und einen Durchmesser von 0,5 cm bis 2,5 cm aufweisen. Jedenfalls kann die Handhabung des Filmverbands in der Unterdruckwundtherapie mit der herstellerseitig vorgesehenen Öffnung verbessert werden, indem der Anwender die Öffnung nicht mehr selbst erzeugen muss und ihm somit ein Arbeitsschritt beim Verbinden der Wunde abgenommen wird.

Ein weiterer Gegenstand der vorliegenden Erfindung ist ein Verbandskit für eine Unterdruckwundtherapie. Der Kit umfasst die folgenden Komponenten:
- einen erfindungsgemäßen Filmverband wie vorangehend beschrieben
- ein Wundfüllmaterial
- einen Saugschlauch zum Verbinden des Filmverbands mit einer Unterdruckquelle
- optional eine Gebrauchsanweisung
Mit dem Kit können dem Anwender auf einfache Weise alle wesentlichen Verbandsmaterialien bereitgestellt werden, die er für die Versorgung einer Wunde im Rahmen einer Unterdruckwundtherapie benötigt.

Der Kit ist bevorzugt steril. Weiterhin kann der Kit auch ohne ein zusätzliches, separat bereitgestelltes Mittel zum Befestigen und/oder Abdichten des Filmverbands am Körper eines Patienten bereitgestellt werden.

Ein weiterer Gegenstand der vorliegenden Erfindung ist ein System für eine Unterdruckwundtherapie. Das System umfasst die folgenden Komponenten:
- einen erfindungsgemäßen Filmverband wie vorangehend beschrieben
- ein Wundfüllmaterial
- eine Unterdruckquelle
- einen Saugschlauch zum Verbinden des Filmverbands mit der Unterdruckquelle.
Die Unterdruckquelle kann einen austauschbaren Behälter zum Sammeln des abgesaugten Wundexsudats umfassen.

Zudem wird ein Verfahren zur Herstellung des erfindungsgemäßen Filmverbands angegeben. Das Verfahren umfasst die nachfolgenden Schritte:
i. Bereitstellen einer ersten, flüssigkeitsundurchlässigen und wasserdampfdurchlässigen Kunststofffolie mit einer Oberseite und einer Unterseite.
ii. Beschichten, zum Beispiel Musterbeschichten oder insbesondere vollflächiges Beschichten, der Unterseite der ersten Kunststofffolie mit einem Acrylatklebstoff, wobei ein erster Verbund erhalten wird.
iii. Bereitstellen einer zweiten Kunststofffolie mit einer Oberseite und einer Unterseite.
iv. Beschichten, insbesondere vollflächiges Beschichten, der Oberseite der zweiten Kunststofffolie mit einem Acrylatklebstoff und der Unterseite der zweiten Kunststofffolie mit einem Silikongel, wobei ein zweiter Verbund erhalten wird.
v. Erzeugen von Perforationen in dem zweiten Verbund insbesondere durch Schneiden oder Stanzen.
vi. In Kontakt bringen des Acrylatklebstoffs des ersten Verbunds mit dem Acrylatklebstoff des zweiten Verbunds, so dass der erste Verbund und der zweite Verbund miteinander verbunden sind.
Dabei sind der erste Verbund und der zweite Verbund im Hinblick auf ihre Größe (Fläche) derart ausgestaltet, dass der erste Verbund den zweiten Verbund überragen und somit einen adhäsiven Rand des Filmverbands wie vorangehend beschrieben ausbilden kann.

Indem in Verfahrensschritt vi. zwei Acrylatklebstoffschichten miteinander in Kontakt gebracht werden, kann eine besonders gute Befestigung des zweiten Verbunds an dem ersten Verbund erzielt werden. Die Gefahr einer Trennung (Delamination) des Filmverbands kann dadurch verringert werden.

Der Filmverband kann in all seinen vorangehend beschriebenen Ausführungsformen für den Kit und das System verwendet und mit dem Verfahren hergestellt werden. Die zusätzlichen Merkmale des Filmverbands in seinen speziellen Ausführungsformen sind daher ebenso im Zusammenhang mit dem Kit, dem System sowie dem Verfahren offenbart.

### Figuren

Mit den nachfolgend beschriebenen, schematischen Figuren soll die Erfindung exemplarisch näher erläutert und veranschaulicht werden. Dabei können in den Figuren ähnliche Strukturelemente mit denselben Bezugszeichen ausgewiesen sein.

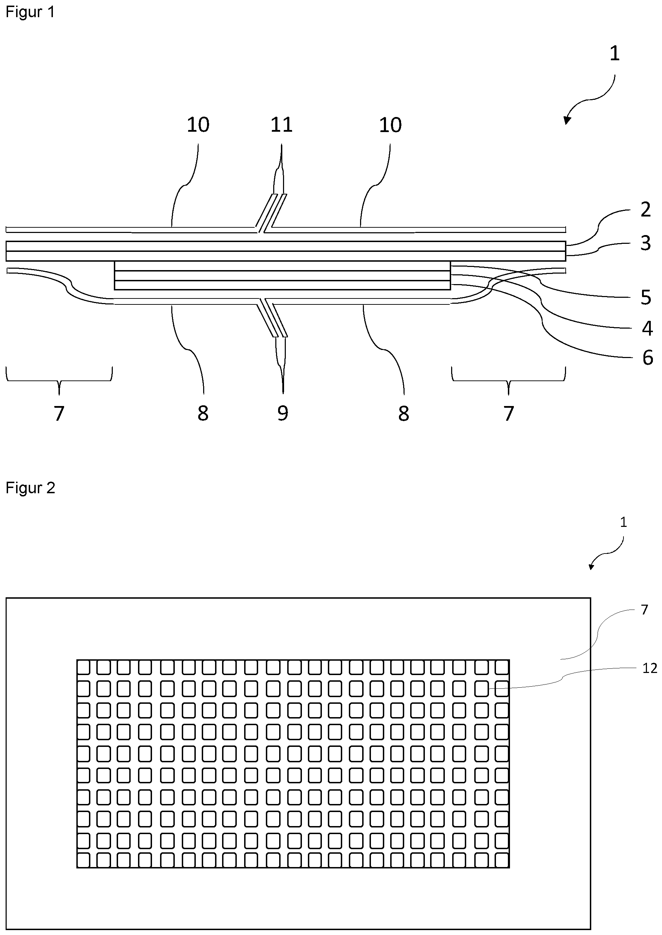

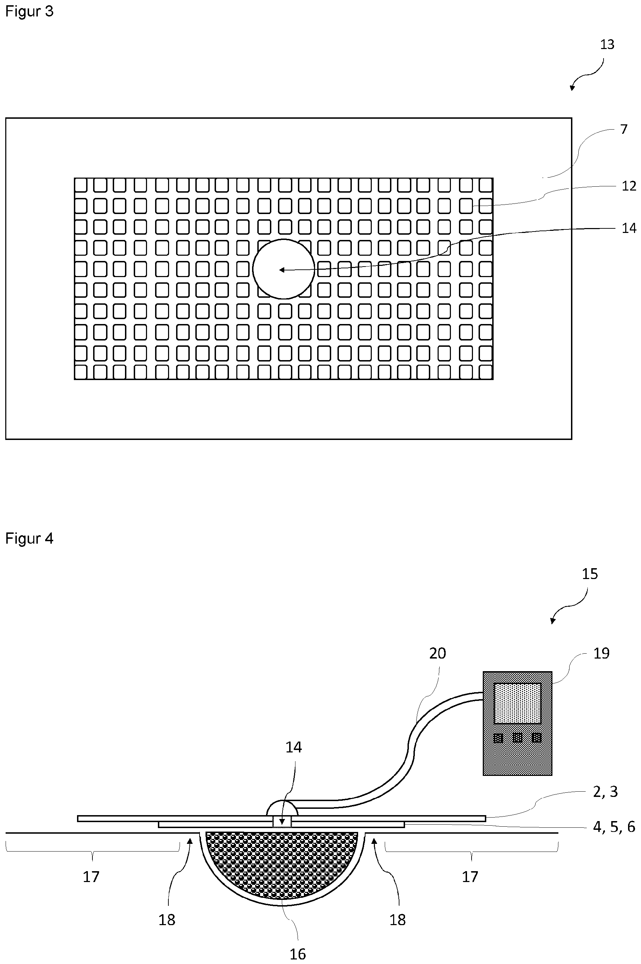

**Figur 1** zeigt einen erfindungsgemäßen Filmverband **1** in einer ersten Ausführungsform in einer seitlichen Schnittansicht. Der Filmverband **1** umfasst eine flüssigkeitsundurchlässige und wasserdampfdurchlässige Kunststofffolie **2** (erste Kunststofffolie). An ihrer Unterseite ist die Kunststofffolie **2** vollflächig mit einem Acrylatklebstoff **3** (erste adhäsive Schicht) beschichtet. An die adhäsiv beschichtete Kunststofffolie **2, 3** (erster Verbund) ist ein dreischichtiger, perforierter Verbund (zweiter Verbund) befestigt. Dieser besteht aus einer Kunststofffolie **4** (zweite Kunststofffolie), welche auf ihrer Oberseite mit einem Acrylatklebstoff **5** (zweite adhäsive Schicht) und auf ihrer Unterseite mit einem Silikongel **6** (dritte adhäsive Schicht) beschichtet ist. Die adhäsive Kunststofffolie **2, 3** überragt den dreischichtigen, perforierten Verbund **4, 5, 6,** so dass der Filmverband einen adhäsiven Rand **7** aufweist. Zudem umfasst der Filmverband **1** ein Applikationssystem. Dieses umfasst eine zweiteilige Schutzfolie **8** mit Grifflaschen **9** sowie eine zweiteilige Stützfolie **10** mit Grifflaschen **11.** Mit den Grifflaschen **9, 11** kann der Anwender das Applikationssystem leichter entfernen, wenn er den Filmverband **1** an der Wundstelle befestigt. Nachdem die Schutz- und Stützfolien **8, 10** vom Filmverband **1** abgezogen wurden und der Filmverband **1** an der Wundstelle bestimmungsgemäß befestigt wurde, bildet die Kunststofffolie **2** die Rückschicht, also die äußerste Schicht des Filmverbands **1.** Der Verbund **4, 5, 6** beziehungsweise dessen Silikongel **6** hat zumindest dann Kontakt mit der Wundoberfläche und bildet folglich eine Wundkontaktschicht, wenn der Filmverband 1 als primäre Wundauflage und nicht als sekundäre Wundauflage (wie in Figur 4 gezeigt) verwendet wird. Da der Filmverband **1** (ohne Applikationssystem) transparent ist, kann der Zustand der Wunde und deren Heilungsverlauf vorteilhafterweise jederzeit leicht festgestellt werden.
**Figur 2** zeigt den Filmverband **1** aus Figur 1 mit Blick auf seine adhäsive Unterseite (ohne Schutzfolien **8**)**.** Zu erkennen ist hier die zentrale Anordnung des silikonisierten Verbunds **4, 5, 6** mit dem umlaufenden adhäsiven Rand **7** sowie die Perforationen **12,** welche den zentral angeordneten Verbund **4, 5, 6** durchdringen. Dabei ist die Anzahl, Größe und Form der Perforationen **12** beispielhaft zu verstehen. Insbesondere können die Perforationen **12** auch rund ausgebildet und kleiner sein. Durch den stärker klebenden Randbereich in Verbindung mit dem schwächer klebenden zentralen Bereich kann der Filmverband **1** wie bereits beschrieben sowohl gut an der Wundstelle haften als auch atraumatische Eigenschaften aufweisen.
**Figur 3** zeigt die Unterseite eines erfindungsgemäßen Filmverbands **13** in einer zweiten Ausführungsform, welche speziell für die Anwendung in der Unterdruckwundtherapie vorgesehen ist. Der Filmverband **13** unterscheidet sich von dem Filmverband **1** lediglich dadurch, dass er zusätzlich eine Öffnung **14** zum Absaugen von Luft im Rahmen der Unterdruckwundtherapie aufweist. Die Öffnung **14** ist mittig im Filmverband **13** eingebracht und im Vergleich zu den Perforationen **12** viel größer ausgebildet. Außerdem durchdringt sie nicht nur den zweiten Verbund **4, 5, 6,** sondern auch den ersten Verbund **2, 3.**

Schließlich ist in **Figur 4** ein System **15** für eine Unterdruckwundtherapie dargestellt. Das System **15** umfasst einen porösen Wundfüller **16,** welcher die Wunde ausfüllt. Der Wundfüller **16,** beispielsweise ein offenzelliger Polyurethanschaumstoff, ist passend für die Wunde zugeschnitten und stellt die primäre Wundauflage mit Wundkontakt dar. Der Wundfüller **16** dient als Unterdruckverteiler und stellt sicher, dass Flüssigkeiten und Gase aus der Wunde abgesaugt werden können. Weiterhin umfasst das System **15** den Filmverband **13** aus Figur 3 als sekundäre Wundabdeckung. Zur Vereinfachung sind einige Schichten des Filmverbands **13** in Figur 4 zusammengefasst worden. Der Filmverband **13** ist derart an der Wundstelle befestigt, dass er die mit dem Wundfüller **16** ausgekleidete Wunde abgesehen von der Öffnung **14** luftdicht versiegelt. Dabei ist der adhäsive Rand **7** an intakter Haut **17** in der Wundumgebung und der silikonisierte, zentrale Bereich zusätzlich am Wundrand **18** sowie dem Wundfüller **16** befestigt. Schließlich umfasst das System **15** noch eine Unterdruckquelle **19** und einen Saugschlauch **20.** Der mit der Unterdruckquelle **19** in Verbindung stehende Saugschlauch **20** ist and der Außenseite des Filmverbands **13** im Bereich der Öffnung **14** luftdicht angeschlossen. Damit kann im Rahmen der Unterdruckwundtherapie ein von der Unterdruckquelle **19** erzeugter Unterdruck an die Wunde weitergegeben und im Wundraum aufrechterhalten werden. Eine Unterdruckwundtherapie mit dem System **15** kann durch den atraumatischen Filmverband **13** insbesondere den Wundrand **18** beim Verbandswechsel schonen.

Die in den Figuren gezeigten Filmverbände haben eine rechteckige Form. Die Filmverbände können jedoch auch eine andere Form haben, zum Beispiel rund oder oval. Ebenso ist es vorgesehen, dass die erfindungsgemäßen Filmverbände nicht nur in verschiedenen Formen, sondern auch in verschiedenen Größen zur Verfügung gestellt werden. Verbände in unterschiedlichen Formen und Größen sind gängige Praxis in der Wundversorgung, damit möglichst für jede Wunde unabhängig davon wo sie sich am Körper des Patienten befindet und unabhängig von ihrer Größe ein passender Verband verfügbar ist und ausgewählt werden kann. Ein Zurechtschneiden eines erfindungsgemäßen Filmverbands ist zwar theoretisch möglich. Allerdings kann dann der adhäsive Rand an der Schnittkante fehlen und die Haftkraft an der Schnittkante zu gering sein. Daher sollte der vorliegende Filmverband nach Möglichkeit nicht geschnitten werden.

## Patentansprüche

1. Filmverband (1, 13) für eine Wundbehandlung, umfassend
i. eine erste, flüssigkeitsundurchlässige und wasserdampfdurchlässige Kunststofffolie (2) mit einer Oberseite und einer Unterseite,
ii. eine erste adhäsive Schicht (3) mit einer Oberseite und einer Unterseite, wobei die erste adhäsive Schicht (3) mit ihrer gesamten Oberseite direkt an der Unterseite der ersten Kunststofffolie (2) angeordnet ist und aus einem Acrylatklebstoff besteht,
iii. eine zweite adhäsive Schicht (5) mit einer Oberseite und einer Unterseite, wobei die zweite adhäsive Schicht (5) mit ihrer gesamten Oberseite direkt an der Unterseite der ersten adhäsiven Schicht (3) angeordnet ist und aus einem Acrylatklebstoff besteht,
iv. eine zweite Kunststofffolie (4) mit einer Oberseite und einer Unterseite, wobei die zweite Kunststofffolie (4) mit ihrer gesamten Oberseite direkt an der Unterseite der zweiten adhäsiven Schicht (5) angeordnet ist, und
v. eine dritte adhäsive Schicht (6) mit einer Oberseite und einer Unterseite, wobei die dritte adhäsive Schicht (6) mit ihrer gesamten Oberseite direkt an der Unterseite der zweiten Kunststofffolie (4) angeordnet ist und aus einem Silikongel besteht,
wobei die erste Kunststofffolie (2) mit der ersten adhäsiven Schicht (3) einen ersten Verbund (2, 3) ausbildet und die zweite Kunststofffolie (4) mit der zweiten und dritten adhäsiven Schicht (5, 6) einen zweiten Verbund (4, 5, 6) ausbildet,
wobei der erste Verbund (2, 3) den zweiten Verbund (4, 5, 6) überragt, so dass der Filmverband (1, 13) einen adhäsiven Rand (7) bestehend aus der ersten Kunststofffolie (2) und der ersten adhäsiven Schicht (3) aufweist, und
wobei der zweite Verbund (4, 5, 6) perforiert ist.

2. Filmverband (1, 13) nach Anspruch 1, wobei die erste und zweite Kunststofffolie (2, 4) sowie die erste bis dritte adhäsive Schicht (3, 5, 6) im Wesentlichen transparent ausgebildet sind.

3. Filmverband (1, 13) nach Anspruch 1 oder 2, wobei die Klebkraft der ersten adhäsiven Schicht (3) mehr als 2,8 N/25 mm, bevorzugt mehr als 3,5 N/25 mm, und besonders bevorzugt mehr als 4 N/25 mm beträgt.

4. Filmverband (1, 13) nach einem der vorangehenden Ansprüche, wobei die Klebkraft der dritten adhäsiven Schicht (6) 1,5 N/25 mm bis 2,7 N/25 mm beträgt.

5. Filmverband (1, 13) nach einem der vorangehenden Ansprüche, wobei der zweite Verbund (4, 5, 6) 30 % bis 90 %, bevorzugt 30 % bis 80 %, mehr bevorzugt 30 % bis 70 % und besonders bevorzugt 50 % bis 70 % einer Unterseite des Filmverbands (1, 13) bildet.

6. Filmverband (1, 13) nach einem der vorangehenden Ansprüche, wobei der Filmverband (1, 13) weiterhin mindestens eine Schutzfolie (8) mit einer Oberseite und einer Unterseite umfasst, wobei die Schutzfolie (8) mit ihrer Oberseite direkt an der Unterseite der ersten adhäsiven Schicht (3) im Bereich des adhäsiven Rands (7) und der dritten adhäsiven Schicht (6) lösbar angeordnet ist.

7. Filmverband (1, 13) nach einem der vorangehenden Ansprüche, wobei der Filmverband (1, 13) weiterhin mindestens eine Stützfolie (10) mit einer Oberseite und einer Unterseite umfasst, wobei die Stützfolie (10) mit ihrer Unterseite direkt an der Oberseite der ersten Kunststofffolie (2) lösbar angeordnet ist.

8. Filmverband (1, 13) nach einem der vorangehenden Ansprüche, wobei der Filmverband (1, 13) aus
i. der ersten Kunststofffolie (2),
ii. der ersten adhäsiven Schicht (3),
iii. der zweiten adhäsiven Schicht (5),
iv. der zweiten Kunststofffolie (4),
v. der dritten adhäsiven Schicht (6),
vi. optional der mindestens einen Schutzfolie (8), und
vii. optional der mindestens einen Stützfolie (10)
besteht.

9. Filmverband (1, 13) nach einem der vorangehenden Ansprüche, wobei der Filmverband (1, 13) derart ausgebildet ist, dass lediglich die erste adhäsive Schicht (3) im Bereich des adhäsiven Rands (7) und die dritte adhäsive Schicht (6) mit einer Haut- oder Wundoberfläche eines Patienten in Kontakt treten kann.

10. Filmverband (1, 13) nach einem der vorangehenden Ansprüche, wobei der Filmverband (1, 13) derart ausgebildet ist, dass die erste adhäsive Schicht (3) nicht in die Perforationen (12) des zweiten Verbunds (4, 5, 6) bis zur Unterseite der dritten adhäsiven Schicht (6) eindringen kann.

11. Filmverband (1, 13) nach einem der vorangehenden Ansprüche, wobei der Filmverband (1, 13) dafür vorgesehen ist ohne ein separat bereitgestelltes Mittel zum Befestigen und/oder Abdichten des Filmverbands (1, 13) am Körper eines Patienten für die Wundbehandlung verwendet zu werden.

12. Filmverband (13) nach einem der vorangehenden Ansprüche, wobei der Filmverband (13) eine Öffnung (14) zum Absaugen von Luft im Rahmen einer Unterdruckwundtherapie aufweist.

13. Kit für eine Unterdruckwundtherapie, umfassend
- einen Filmverband (1, 13) nach einem der vorangehenden Ansprüche,
- ein Wundfüllmaterial,
- einen Saugschlauch zum Verbinden des Filmverbands mit einer Unterdruckquelle, und
- optional eine Gebrauchsanweisung.

14. System (15) für eine Unterdruckwundtherapie, umfassend
- einen Filmverband (13) nach einem der Ansprüche 1 bis 12,
- ein Wundfüllmaterial (16),
- eine Unterdruckquelle (19), und
- einen Saugschlauch (20) zum Verbinden des Filmverbands (13) mit der Unterdruckquelle (19).

15. Verfahren zur Herstellung eines Filmverbands (1, 13) nach einem der Ansprüche 1 bis 12, wobei das Verfahren die folgenden Schritte umfasst:
i. Bereitstellen einer ersten, flüssigkeitsundurchlässigen und wasserdampfdurchlässigen Kunststofffolie (2) mit einer Oberseite und einer Unterseite,
ii. Beschichten, zum Beispiel Musterbeschichten oder insbesondere vollflächiges Beschichten, der Unterseite der ersten Kunststofffolie (2) mit einem Acrylatklebstoff (3), wobei ein erster Verbund (2, 3) erhalten wird,
iii. Bereitstellen einer zweiten Kunststofffolie (4) mit einer Oberseite und einer Unterseite,
iv. Beschichten, insbesondere vollflächiges Beschichten, der Oberseite der zweiten Kunststofffolie (4) mit einem Acrylatklebstoff (5) und der Unterseite der zweiten Kunststofffolie (4) mit einem Silikongel (6), wobei ein zweiter Verbund (4, 5, 6) erhalten wird,
v. Erzeugen von Perforationen (12) in dem zweiten Verbund (4, 5, 6) insbesondere durch Schneiden oder Stanzen,
vi. in Kontakt bringen des Acrylatklebstoffs (3) des ersten Verbunds (2, 3) mit dem Acrylatklebstoff (5) des zweiten Verbunds (4, 5, 6), so dass der erste Verbund (2, 3) und der zweite Verbund (4, 5, 6) miteinander verbunden sind,
wobei der erste Verbund (2, 3) und der zweite Verbund (4, 5, 6) derart ausgestaltet sind, dass der erste Verbund (2, 3) den zweiten Verbund (4, 5, 6) überragen und somit einen adhäsiven Rand (7) des Filmverbands (1, 13) ausbilden kann.
